# EUROPEAN PATENT APPLICATION

(11) **EP 2 808 337 A1**
(43) Date of publication of application: **03.12.2014**
(21) Application number: 14150858.0
(22) Date of filing: 12.01.2014
(51) Int. Cl.: C07F 9/655, A61K 31/665, A61P 35/00

(54) **(E)-3-Aryl-3-oxoprop-1-enyl-2-phosphonic acid and its derivatives, methods for their preparation and their use**

(30) Priority: 28.05.2013 PL 40411213
(71) Applicant: Centrum Badan Molekularnych I Makromolekularnych Pan, 90-363 Lodz (PL)
(72) Inventor: Balczewski, Piotr, 91-225 Lódz (PL)
(74) Representative: Brodowska, Iwona

(57) **Abstract**

The present invention is (E)-3-aryl-3-oxoprop-1-enyl-2-phosphonic acid and its derivatives in the form of esters or salts, process for their preparation and use as compounds having ability to interact with nucleic acids, including DNA intercalating properties and antitumor activity.

## Description

### (E)-3-Aryl-3-oxoprop-1-enyl-2-phosphonic acid and its derivatives, methods for their preparation and their use

The present invention is (E)-3-aryl-3-oxoprop-1-enyl-2-phosphonic acid and its derivatives, optionally in the form of esters or salts, methods for their preparation and use as compounds having ability to interact with nucleic acids, especially those with DNA intercalating properties and antitumor activity. These compounds are represented by the formula 1, wherein:

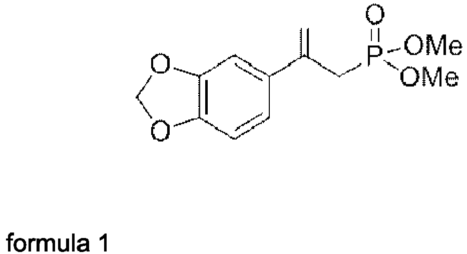

Ar - represents an aryl group selected from the group including phenyl, benzo[d][1,3]dioxolane, 3,5-dimethoxyphenyl, 3,4,5-trimethoxyphenyl group, unsubstituted or substituted cyclic alkoxy group, especially a 1,3-dioxamethylene - OCH2O-, or acyclic one, especially methoxy MeO, ethoxy EtO, propoxy PrO or butoxy BuO, and a heteroaryl group, selected from the group containing thienyl, pyrrolyl, furyl or pyridyl group.

**R1**, **R2** - are the same or different and represent the group -OH, -OMetal, wherein the Metal is Li, Na, K, Zn, Mg, or an -O-alkyl group, wherein alkyl contains from 1 to 6 carbon atoms,

### State of the art

Polycyclic fused aromatic hydrocarbons and heteroaromatics are commonly known as compounds interacting with DNA, primarily on the basis of intercalation.

Structures of the formula 1, possessing pyrenyl group are not known in the literature and, therefore, their properties have not been investigated so far.

Mahadevan and Parsons1 studied the effect of synthetic mixtures of polycyclic aromatic hydrocarbons (PAH) on cell lines of the breast cancer MCF-7 and analyzed the formation of PAH-DNA adduct and the induction of cytochrome enzymes P450 (CYP). Both, a group considered as noncarcinogenic (including pyrene) and a group of mildly carcinogenic, polycyclic aromatic hydrocarbons induce enzymes CYP1A1 and CYP1 B1 in tumor cell lines MCF-7 and affect the binding to DNA.

Lisby and Schneider2 used pyrenyl derivatives linked to the phosphate group of nucleotide, as intercalators, in a new method of analysis and detection of short sequences of oligonucleotides that allows for easy identification of a single base in the system and mismatch correction.

Pyrene derivatives were also applied as intercalators in the PCR method (Polymerase Chain Reaction) which is used to amplify strands of DNA or RNA selected for analysis.3, 4

In the studies of gene silencing with the use of chemically modified nucleic acids, pyrenyl group was used, as a hydrophobic part of the modified oligonucleotide.5

Pyrene and its derivatives such as pyrenyl butyrate, alkylated pyrene as well as other pyrenyl derivatives containing free amino and carboxyl groups, albumin- pyrene analogs and conjugate PEG-pyrene [poly(ethylene glycol)-pyrene], after coupling with the peptide factor, were used in the diagnosis and treatment of neurological disorders.6

Bossman and Troyer7 proposed a new type of diagnostic nano-tests for detecting tumor cells in mammals, by means of which the level of protease activity is determined. These nano-tests are constructed on the basis of the combination of two chromophoric molecules: coumarin and pyrene.

Lyles8, using a planar structure of xanthine, patented a method that allows the suppression of nucleic acid binding by the polycyclic aromatic hydrocarbons, including pyrene and benzo[a]pyrene, and the removal of intercalating molecule due to creating a competitive PAH-xanthine complex.

Derivatives of 3'-pyrenyl ribose, through fluorescent properties, have found application for quantitative and qualitative screening in medicine.9,10

Pyrene fluorescent tags connected to the individual components of the mixtures were used for identification of small organic molecules, such as polymers, peptides, sugars and nucleic acids, based on measurement of different fluorescence lifetimes.11 Inhibiting the activity of the enzyme DHFR (dihydrofolate reductase), responsible for the conversion of DH2-folate to TH4-folate delivered to all cells in the body, can be treated by administration to the patient a therapeutic amount of a mixture of nonaliphatic hydrocarbons (including pyrene).12

Pyrene was also used in studies on polymeric drug carriers13, wherein it served as a hydrophobic drug trapped in polymeric micelle.

### Summary of the Invention

Compounds of the general the formula 1, 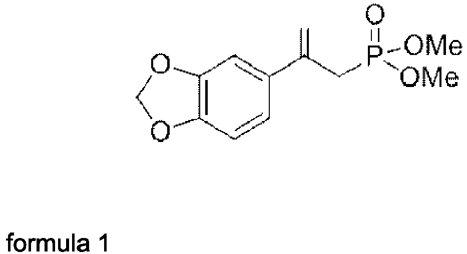 wherein:
Ar - represents an aryl group selected from the group involving phenyl, benzo[d][1,3]dioxolane, 3,5-dimethoxyphenyl, 3,4,5-trimethoxyphenyl group, unsubstituted or substituted cyclic alkoxy group, especially a 1,3-dioxamethylene - OCH2O-, or acyclic one, especially methoxy MeO, ethoxy EtO, propoxy PrO or butoxy BuO, and a heteroaryl group, selected from the group involving thienyl, pyrrolyl, furyl or pyridyl group.

**R1**, **R2** - are the same or different and represent the group -OH, -OMetal, wherein the Metal is Li, Na, K, Zn, Mg, or a group -O-alkyl, wherein alkyl contains from 1 to 6 carbon atoms.

According to the invention, suitable compounds are represented by the formulae **1a, 1b** and **1c**.

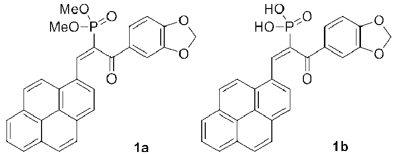

The compounds of the formulae 1a and 1b due to the interaction with deoxyribonucleic acid (DNA) on the basis of intercalation or similar physical and chemical interactions, may be used for the production of pharmaceuticals applicable in the anticancer therapies. Compounds 1c, in the form of water soluble salts, may be convenient pharmacological formula for the compounds of 1a and 1 b.

Compounds of the general formula 1 were synthesized under mild conditions, and the reaction route is shown in Scheme I.

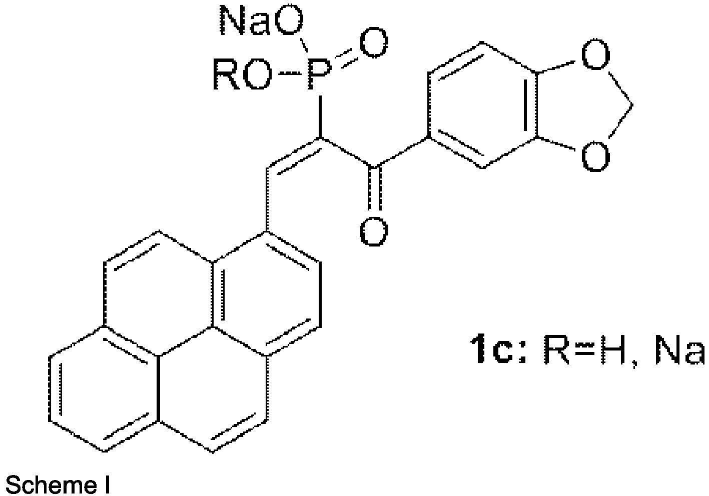

The invention relates also to the method of preparation of compounds of the general formula **1**, according to the invention, based on the fact that the β- ketophosphonate **2** is subjected to a condensation reaction with stoichiometric amount of pyrenyl aldehyde in the presence of catalytic amount of piperidine and in an organic solvent, preferably toluene, and then the hydrolysis of the obtained derivative **1a** (1: R1=R2=OMe) is carried out in the presence of trimethylsilyl bromide, TMSBr, in an organic solvent, preferably methylene chloride and methanol, to afford (Z)-3-(benzo[d][1,3]dioxol-5-yl)-1-(pyrene-1-yl)-3-oxoprop-1-en-2-ylphosphonic acid **1b** (1: R1=R2=OH). As a result of neutralization of a suspension of the compound **1b** in water, with an aqueous NaOH solution, aqueous solution of **1c** in the form of mono (R=H) or disalts (R=Na) can be obtained. (Scheme I).

The subject of the invention is also the application of compounds of general formula 1 for production of pharmaceuticals used in the antitumor therapy.

Especially preferred is the use of compounds of the formula **1a** and **1b** for the preparation of pharmaceuticals applicable in the antitumor therapies, interacting with deoxyribonucleic acid (DNA) on the basis of intercalation and/or similar physicochemical interactions, while the compounds **1c**, in the form of water-soluble salts, constitute a convenient pharmacological formula for application of the compounds **1a** and **1b**.

The following are examples of preparation of the compounds according to the invention and their use.

### Example I. Procedure for the synthesis of the compound of the formula 1a:

To a solution of β-ketophosphonate 2 (1.314 g, 4.83 mmol) in toluene a catalytic amount of piperidine was added (0.024 g, 0.24 mmol) and stoichiometric amount of pyrenyl aldehyde (1.110 g, 4.83 mmol). The resulting solution was heated with the use of an Dean-Stark apparatus for 20 hours. The solvent was evaporated and the residue was purified by column chromatography on a silica gel. The compound 1a was obtained, as a yellow solid with a melting point of 161-162°C.

### Example II. The procedure for the synthesis of the compound of the formula 1b:

To a solution of the compound **1a** (0.30 g, 4.11 mmol) in anhydrous methylene chloride, trimethylsilyl bromide TMSBr was added (41.10 mmol) and the resulting mixture was heated for 3 hours and then methanol was added and it was heated for additional one hour. The solvents were evaporated and the residue was dried under reduced pressure (oil pump) to afford the acid **1b** in high yield and purity up to 100%. The acid **1b** can be purified in a standard manner by the alkaline extraction, transforming it into the disodium salt **1c**, extraction of impurities using an organic solvent, acidification with an aqueous solution of HCl, salting out by solid NaCl followed by extraction of the acid **1b** with diethyl ether.

### Example III. The procedure for the synthesis of the compound of formula 1 c:

To a suspension of the acid **1b** in water, an aqueous alkali was added, preferably NaOH, in an amount of one (mono salt) or two (disalt) equivalents until complete dissolution of the suspension, to yield aqueous solutions of sodium salts **1c**.

Proceeding as in the above examples, the compounds **1a** and **1b** were prepared, and their spectroscopic data are summarized in Table 1.

**Table 1**

| Comp ound | 1HNMR[ppm] 200MHz,CDCl3 | 13CNMR[ppm] 50MHz,CDCl3 | MS-EI (70eV, %) |
|---|---|---|---|
| **1a** | 3.87 (s, 3H, P(O)(OCH3), 3.93 | 52.12(s,OCH3) | 484(M+,48), |
| | (s, 3H, P(O)(OCH3), 5.80 (s, 2H, OCH2O), 6.42 (d, | 100.49(s,OCH2O), | 375(M+- |
| | J3HH=8.1 Hz, 1H, ArH) 7.35- | 106.48, 106.96, 121.68, | P(O)(OCH3)2, |
| | 7.44 (m, 2H, ArH), 7.87-8.06 (m, 5H, ArH), 8.06-8.26 (m, | 123.29, 124.77, 124.82, | 36), 374 (100), |
| | 3H, ArH) 8.41 (s, 1H, ArH), | 125.07, 125.51, 125.93, | |
| | 8.87 (d, J3PH=24.7 Hz,1H, HC=CP | 127.35, 129.37, 187.63 | |

**Table 0002**

| | | | |
|---|---|---|---|
| **1b** | 5.66 (s, 2H, OCH2O), 6.31 (d, | | 457.2 (M+ + 1,2), 377 (M+- |
| | J3HH=8.1 Hz, 1 H, ArH) 7.25 (s, 1H, ArH), 7.40-7.44 (m, 1H, | | |
| | ArH), 7.65-8.29 (m, 11H, ArH) | | P(O)(OH)2, 93) |
| | 8.82 (d, J3PH=25.8 Hz,1H, | | |
| | HC=CP) | | |

Especially preferred, according to the invention, are combinations represented by the formulae **1a**, **1b** and **1c**.

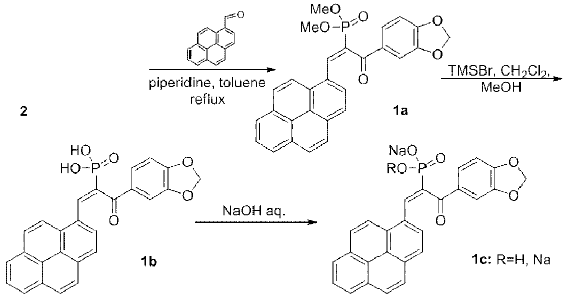

### Example IV.

The impact of compounds **1a** and **1b** has been tested on the digestion of DNA plasmid pcDNA 3.1 HisC (8.2 kb) with restriction enzyme Bam H1.

1. For this purpose, pcDNA 3.1 HisC plasmid DNA (0.6 µg) was dissolved in 1×BamH1 buffer and incubated overnight at 37°C with the following reference or test compounds:

a. Daunorubicin - a reference compound used in the treatment of acute leukemias. It causes cell death through binding to DNA helix and preventing its replication

### Test compound 1a,

### Test compound 1b

The concentration of the reference and test compounds in each sample was 2 or 20 µM.

2. After overnight incubation, the reaction mixture was digested with BamH1 restriction enzyme (2U/sample) for 3 hours at 37°C. The total volume of the samples was 10 µL.

3. The reaction products were applied on 1% agarose gel and electrophoresis was performed in TAE buffer.

Detailed samples composition is presented in Table 2.

**Table 2**

| **Sample No** | **Plasmid** | **10xbuffer for Bam H1** | **DMSO** | **Compound (stock conc. 200 µM or 20 µM)** | **Water** | **Enzyme** |
|---|---|---|---|---|---|---|
| | **(0.6mg/ ml)** | | | | | **Bam H1 (2U/µl)** |
| 1control | 1 µl | 1 µl | 1 µl | - | 7µl | - |
| 2control | 1 µl | 1 µl | 1 µl | - | 6µl | 1 µl |
| 3 | 1 µl | 1 µl | - | DAUNO | 6µl | 1 µl |
| | | | | 1µl 200µM | | |
| 4 | 1 µl | 1 µl | - | DAUNO | 6µl | 1 µl |
| | | | | 1µl 20µM | | |
| 5 | 1 µl | 1 µl | - | **1a** | 6µl | 1 µl |
| | | | | 1µl 200µM | | |
| 6 | 1 µl | 1 µl | - | **1a** | 6µl | 1 µl |
| | | | | 1µl 20µM | | |
| 7 | 1 µl | 1 µl | - | **1b** | 6µl | 1 µl |
| | | | | 1 µl 200µM | | |
| 8 | 1µl | 1 µl | - | **1b** | 6µl | 1 µl |
| | | | | 1 pl 20pM | | |

The test compounds 1a and 1b as well as daunorubicin interact with plasmid DNA (pcDNA 3.1 HisC) increasing its resistance to digestion by the endonucleolytic enzyme Bam H1 (not shown). No significant differences between the ester **1a** and the acid **1b** were observed in this case.

### Example V.

The ability of the test compounds **1a** and **1b** to interact with DNA was tested by the circular dichroism (CD) technique.

1. For this purpose, samples were prepared in PBS buffer containing: 2pM of synthetic DNA duplex (23 nt- 5'TCT TCA AGA ATT CAG GTC CTG AT 3') or DNA of bovine thymus (0.2 mg/ml) and test compounds at a concentration of 20µM or 40µM for synthetic DNA duplex sample and thymus DNA sample, respectively. The samples contained 0.2% or 0.4% DMSO (for synthetic DNA duplex sample and thymus DNA sample, respectively), which is a solvent of the test compounds.

2. CD spectra (in the range from 200 nm to 320 nm) were recorded immediately after addition of the test compounds as well as after 1.5 h incubation. The measurement was carried out at 23°C in a quartz cuvette (optical path length 0.5 cm) using a spectroscope CD6 (Jobin-Yvon, France).

3. CD spectra of DNA incubated with the test compounds were compared with the control spectrum (spectrum was recorded for a sample of DNA (2µM or 0.2 mg/ml) in the presence of DMSO (final concentration of 0.2% or 0.4%).

CD spectra of the duplex DNA (2µM) immediately after addition of the test compounds (the DS.328 compound **1a**, the DS.328K compound **1b**) are shown on the graph, Figure 1.

CD spectra of the DNA duplex (2µM) after 1 hour incubation with test compounds (the DS.328 compound **1a**, the DS.328K compound **1b**) are shown on the graph, Figure 2.

CD spectra of the DNA from thymus (0.2 mg/ml) immediately after addition of the test compounds (the DS.328 compound **1a**, the DS.328K compound **1b**) are shown on the graph, Figure 3.

Daunorubicin, a very well-known DNA intercalator interacts with DNA immediately after addition, changing significantly its structure as compared to the control (duplex DNA + DMSO). For the other two compounds: the DS.328 compound **1a** and the DS328K compound **1b**, in the course of their CD spectra one can also observe changes compared to the control sample, which suggest that these compounds may interact with DNA (especially in the range of 220-280 nm).

CD spectra of the DNA duplex recorded after 1.5 hour of incubation with the test compounds, show a much weaker interaction with DNA, what suggests that prolongation of incubation with the test compounds does not affect its interaction with DNA.

The obtained CD results allow to conclude that the test compounds: the DS.328 compound **1a** and the DS.328K compound **1b** can interact with the DNA either on the basis of intercalation or other physicochemical interactions. This is further confirmed by increased resistance of plasmid DNA against endonuclease BamH1 digestion in the presence of test compounds **1a** and **1b**.

Literature

Mahadevan B., Parsons H., Musafia T., Sharma A. K., Amin S., Pereira C., Baird

W. M., Enviromental and Molecular Mutagenesis, 2004, 44, 99-107

Lisby, J. G., Schneider, U. V., Mikkelsen, . D., 2012, WO 2012/055409 A1 3. Schneider, V. U., Johnk, N., Lisby, G. J., 2012, WO 2012/055408 A1

Heindl, D., Ankenbauer, W., Laue, F., 2009, EP 2103693 A1

Khvorova, A., Salomon, W., Kamens, J., Samarsky, D., Woolf, T., Cardia, J., 2010, WO 2010/033247 A2

Wegrzyn, R., Nyborg, A., Duan, D. R., Rudolph, A., 2009, WO 2009/117041 A2 7. Bossman, S. H., Troyer, D. L., Basel, M. T., 2009, WO 2009/111470 A2

8. Lyles, M. B., 2002, WO 02/45707 A2

9. Kool, E. T., Wilson, J. N., Dai, N., 2010, US 2010/0129820 A1

10. IE 010698 "Fluorescent dyed adhesive for bonding various components in a medical devices"

11. Sojka, F. M., Ortega, L., 2004, US 2004/0052742 A1

12. Jensen, M., Parce, W. J., 2002, US 6447724 B1

13. Buck, C. J., 2002, US 3337337 B1

Yokoyama, M., Sakurai, Y., Okano, T., Kataoka, K., 1993, 0583955 A2

CD spectra of the DNA duplex (2µM) immediately after the addition of the

test compounds (the DS.328 compound **1a**, the DS.328K compound **1b**, 20µM with DMSO (0.2%)). **fig. 1** 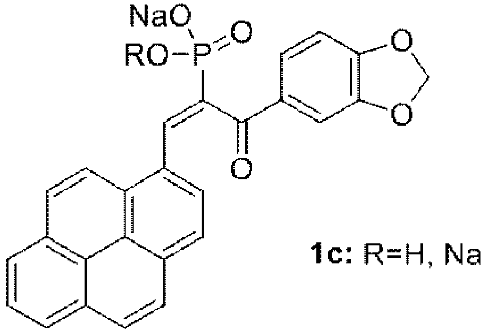 CD spectra of the duplex DNA (2µM) after 1.5 hour of incubation with the test compounds (the DS.328 compound **1a**, the DS.328K compound **1b**, 20µM with DMSO (0.2%)). **fig. 2** 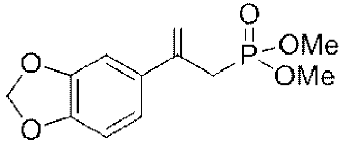 CD spectra of the DNA from thymus (0.2 mg/mL) immediately after addition of the test compounds (the DS.328 compound **1a**, the DS.328K compound **1b**, 40µM with DMSO (0.4%). **fig. 3**

## Claims

1. Compounds of the general formula **1**, 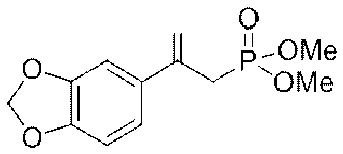 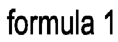 in which: **Ar** - represents an aryl group selected from the group involving phenyl, benzo[d][1,3]dioxolane, 3,5-dimethoxyphenyl, 3,4,5-trimethoxyphenyl group, unsubstituted or substituted cyclic alkoxy group, especially a 1,3-dioxamethylene - OCH2O-, or acyclic one, especially methoxy MeO, ethoxy EtO, propoxy PrO or butoxy BuO, and a heteroaryl group, selected from the group involving thienyl, pyrrolyl, furyl or pyridyl group, **R1**, **R2** - are the same or different and represent the group -OH, -OMetal, wherein the Metal is Li, Na, K, Zn, Mg, or a group -O-alkyl, wherein alkyl contains from 1 to 6 carbon atoms.

2. Compounds according to claim 1, **characterized in that** they are presented by formulae **1a, 1b** and **1c**.

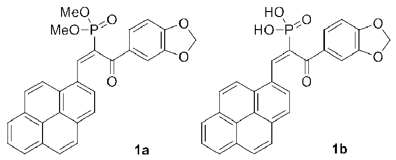

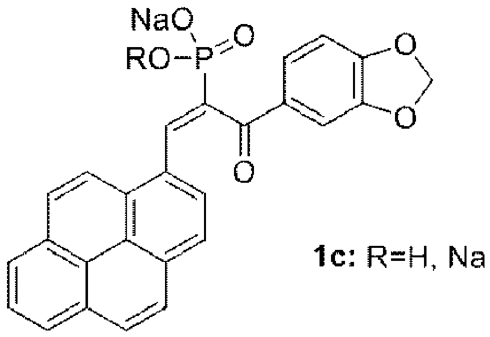

3. A method for the preparation of (E)-3-aryl-3-oxoprop-1-enyl-2-phosphonic acid and its derivatives of general formula 1, 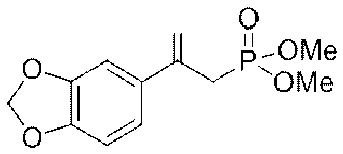 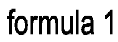 in which: **Ar** - represents an aryl group selected from the group involving phenyl, benzo[d][1,3]dioxolane, 3,5-dimethoxyphenyl, 3,4,5-trimethoxyphenyl group, unsubstituted or substituted cyclic alkoxy group, especially a 1,3-dioxamethylene - OCH2O-, or acyclic, especially methoxy MeO, ethoxy EtO, propoxy PrO or butoxy BuO, and a heteroaryl group, selected from the group involving thienyl, pyrrole, furyl or pyridine group, **R1**, **R2** - are the same or different and represent the group -OH, -OMetal, wherein the metal is Li, Na, K, Zn, Mg, or a group -O-alkyl, wherein alkyl contains from 1 to 6 carbon atoms, is subjected to a condensation reaction with an equivalent amount of pyrenyl aldehyde in the presence of catalytic amount of piperidine and in an organic solvent, preferably toluene, and then is carried out the hydrolysis of the obtained derivative **1a** (1: R1=R2=OMe) in the presence of trimethylsilyl bromide, TMSBr, in an organic solvent, preferably methylene chloride and methanol, to afford (Z)-3-(benzo[d][1,3]dioxol-5-yl)-1-(pyrene-1-yl)-3-oxoprop-1-en-2-ylphosphonic acid of the formula **1b**, R1=R2=OH, and as a result of neutralization a suspension of the compound **1b** in water, with an aqueous NaOH solution, aqueous solution of mono (R=H) or a disalts (R=Na) of the formula **1c** is obtained, as shown in Scheme **I**.

4. A method according to claim 3, **characterized in that** the ester of phosphonic acid of the formula **1**, R1=R2=OMe, is obtained by condensation of a pyrenyl aldehyde with β-ketophosphonate, in which substituents are as defined above, in an alkaline environment, at the boiling point of the organic solvent.

5. A method according to claim 3, **characterized in that** the phosphonic acid of formula **1**, R1=R2=OH, is prepared by hydrolysis of the ester of phosphonic acid of the formula **1**, where R1=R2=OMe, in an organic solvent.

6. A method according to claim 3, **characterized in that** the phosphonic acid salts of the formula **1**, R1=H, R2=ONa, R1=R2=ONa, are obtained by neutralization of the phosphonic acid with alkali (**1**: R1=R2=OH) in which the substituents are as defined above.

7. The use of compounds according to claim 1, for preparation of pharmaceutical compositions containing agents interacting with the DNA, in antitumor therapies.

8. The use according to claim 7, **characterized in that** the production of pharmaceuticals in antitumor therapies, soluble salts of the formula **1c** are used, as a convenient pharmacological formula for the compounds **1a** and **1b**.
